# EUROPEAN PATENT SPECIFICATION

(11) **EP 2 416 740 B1**
(45) Date of publication and mention of the grant of the patent: **30.11.2016**
(21) Application number: 10711772.3
(22) Date of filing: 12.03.2010
(51) Int. Cl.: A61F 2/38

(54) **DEVICE FOR BALANCING A PROSTHETIC IMPLANT, PARTICULARLY FOR A KNEE PROSTHETIC IMPLANT, AND RELEVANT KIT**
GERÄT ZUM ABGLEICHEN EINES PROTHETISCHEN IMPLANTATS, VORALLEM EINER KNIEPROTHESE, UND DAZUGEHÖRIGER BAUSATZ
DISPOSITIF POUR BALANCER UN IMPLANT PROTHÉTIQUE, EN PARTICULIER UN PROTHÈSE DE GENOU, ET KIT PERTINANT

(30) Priority: 06.04.2009 IT RM20090159
(43) Date of publication of application: 15.02.2012
(73) Proprietor: Ferrari Massimo&C.S.a.s., 26100 Cremona (IT)
(72) Inventor: FERRARI, Massimo, 26100 Cremona (IT); GREPPI, Giovanni, 20154 Milano (IT)
(74) Representative: Tiburzi, Andrea
(86) International application number: PCT/IT2010/000107
(87) International publication number: WO 2010/116394

(56) References cited:
- EP-A- 0 903 125
- WO-A-2008/024836
- DE-U1- 9 104 680
- US-A- 5 480 446
- US-A1- 2003 187 510
- US-A1- 2007 239 165

## Description

The present invention relates to a device for balancing a prosthetic implant, particularly for a knee prosthetic implant, and relevant kit.

More specifically, the invention relates to a device for obtaining by surgery a proper balancing of knee articulation.

As it is well known, for surgical techniques, and particularly for knee articulation surgical techniques, a primary goal is that of obtaining during surgery a proper balancing of the articulation, consisting in obtaining an implant seat the volume of which, defined by extension and flexion femur and tibial cutting, can be substantially compared with a symmetric dimensional equivalence defined by capsule-ligament structures.

However, different limits exist to obtain the above goal.

A first limit is comprised of modification of standard capsule-ligament symmetry caused by arthrosis degeneration of articulation ends. Many times, following wear of articulation interfaces and to a consequent loss of cartilaginous and bone substance, after some time, there are modifications of articulation volumes creating articulation axial deviations, with consequent retractions and pathological slack of the capsule - ligament structures of articular kinematic.

Etiopathology which is the basis of degeneration of articular ends can be further attributed to situations by which a primitive morphologic anomaly of epiphysis or their subsequent alteration, consequent to traumatic events or to previous surgical interventions for realignment of skeletal segments, modifies standard articular relationships.

A second limit is represented by surgical reproducibility. When defining spatial orientation of guides to carry out bone cuts necessary to the prosthesis implant, surgeon must necessarily rely on subjective evaluations, since presence of soft tissues hinders a perfect axial identification of skeletal segments. Even use of sophisticated orientation systems of cutting guides, such as modern navigation systems, has reduced, but not eliminated, the above problem.

A third limit is represented by continuity dimensional solution among the different prosthetic sizes of the femur component along sagittal plane, usually between 2 and 4 millimetres.

At present, different surgical techniques exist in order to obtain a proper ligament balancing.

Some of them provide first of all obtainment of balancing under flexion, besides the extension balancing.

In any case, it is well grounded opinion that orientation of tibial osteotomy must be close to orthogonality along coronal plane of mechanical tibial axis in order to prevent over-stressing polyethylene comprising articular interface material which is universally employed for knee prosthesis.

Orientation of tibial cut along coronal plane thus represent an important factor, making a symmetric action between flexion and extension.

As already said, obtaining the proper orientation is subjected to angular errors due to not perfect surgical reproducibility of its execution, both along coronal plane and sagittal plane. In fact, a difference up to 3 degrees can be difficulty appreciated during the surgical intervention, and statistically represents a frequent event.

A difference with respect to orthogonality along coronal plane of the tibial osteotomic plane further conditions orientation of femur rear osteotomic plane that should be parallel to the same. Also when defining orientation of the latter it is possible making an angle error. A possible consequence is due to synergy between angular tibial and femur errors trying to obtain asymmetrical spaces, with unavoidable consequence that said spaces are not balanced.

Furthermore, according to design of prosthetic implant, tibial osteotomy along sagittal plane can provide or not a rear inclination (slope). Even reproduction of said inclination can be subjected to error. An error of its angular orientation with respect to the set angle can dimensionally influence flexion space, increasing it in case of a too large inclination or reducing the same in case of defect.

Spatial positioning of condyle rear cut and its angular orientation should be parallel with respect to tibial osteotomy in order to realise a substantially rectangular space, corresponding to symmetry of prosthetic volumes. Said symmetry is usually obtained by angularly rotating more that desired the cutting plane with respect to the condyle dorsal plane and translating its level in a skull-caudal plane, varying entity of bone cutting with respect to thickness of prosthetic component.

Techniques employed at present in knee articulation surgery substantially provide three different approaches.

First one provides an orientation of cutting plane with reference to femur anatomical parameters (condyle dorsal plane, trans-epicondyle axis, Whiteside Line) determined regardless orientation of tibial cutting.

Second one provides dynamic distraction along tibial plane of femur cutting instruments rotating about a central endo-medullary pin, placed in correspondence of the anatomic axis.

Third one provides using additional thicknesses on a symmetric spacer in order to determine column variations of lateral portion with respect to the medial one.

In the first case, identification of anatomic parameters is subjected to subjective errors by the surgery when evaluating angular position (trans-epicondyle axis and Whiteside Line) or to not determined correspondence of variation between condyle dorsal plane and tibial osteotomic plane.

In both cases, evaluation of amount of cutting is left to experience and ability of the surgeon. Even a two degree error when realising tibial osteotomy (two degrees represent error margin of a known navigation system) can add to an equivalent error when defining orientation of rear osteotomy thus generating compensation hypothesis, substantial neutrality or worsening of tibial error, thus not obtaining parallelism.

Flexion instability can be difficulty corrected by intervening on surgical release of capsule-ligament structures, without any consequence of extension balancing already obtained.

In the second case, orientation of cut and its amount can be conditioned by anatomic structures, such as patellar tendon, which interposes between instruments, thus causing not negligible evaluation errors.

In the third case, use of additional thicknesses on a symmetric spacer does not permit an evaluation correlated between reduction of medial rear cutting and increase of lateral rear cutting along with an angular variation of the osteotomic plane.

The patent applications WO2008/024836 and EP903125 describe a system for adjusting a prosthesis according to the preamble of claim 1.

The relevant prior art includes patent applications EP903125, US2003/187510, US5480446 and DE9104680, which describe prostheses for knee.

Another relevant document of the prior art is the patent application US2007/239165 which discloses a device for balancing a prosthetic implant for a patient.

In view of the above, it is object of the present invention that of overcoming the above mentioned limits.

The present invention is defined by the features of claim 1.

Always according to the invention, said device can be provided laterally with a shaped portion, wherein the rear cruciate ligament of a knee of said patient is placeable.

Still according to the invention, said central body can be provided with one or more seat and said coupling means can comprise one or more magnets, that can be housed in said seats, for maintaining coupled the assembly comprised of said base element, said central body and said upper element.

Furthermore, according to the invention, said central body has a channel and a pair of anchoring holes placed on said upper side, and a threaded hole, placed on the lateral rim, so that a grasping tool provided with pins can be inserted in said channel, coupled to said threaded hole and hooked to said anchoring holes by means of said pins.

Advantageously according to the invention, said upper element can be made in such way that said upper surface is tilted with respect to said lower surface to said adjustment angle.

Always according to the invention, said upper element can be plate-shaped and can have an "8"- shape.

Still according to the invention, said central body and said base element can have substantially the same surface extent.

Furthermore according to the invention,8 said adjustment angle can be comprised between 0°and 9°.

Always according to the invention,, a kit can comprise a grasping tool provided with pins, said grasping tool being couplable with said central element by insertion in said channel, by screwing with said threaded hole and by insertion of said pins in said anchoring holes.

The present invention will be now described, for illustrative but not limitative purposes, according to its preferred embodiments, with particular reference to the figure of the enclosed drawings, wherein:
figure 1 shows a front view of a device for balancing a prosthetic implant according to the present invention;
figure 2 shows a plan view of device of figure 1;
figure 3 shows a section view taken along line B-B of device of figure 1;
figure 4 shows a section view taken along line A-A of device of figure 1;
figure 5 is an exploded view of the device for balancing a prosthetic implant according to the present invention;
figure 6 is a view from below of a central body of the device for balancing a prosthetic implant according to the present invention; and
figure 7 shows a balancing device according to the present invention in a use position.

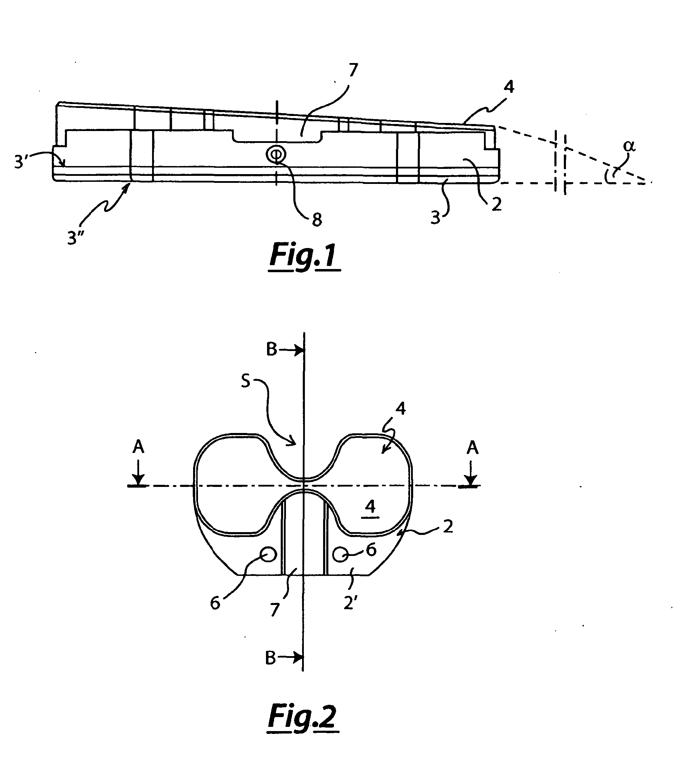

Making reference to figures 1 - 3, it is observed the device 1 for balancing a prosthetic implant according to the present invention.

Said device 1 comprises a central body 2, placed between a base element 3, on the lower portion, and an upper element 4, suitable to obtain an implant seat between the two volumes, defined by extension and flexion femur and tibial cutting, permitting a proper articular balancing.

Said central body 2 has a plate shape with an upper face 2' and a lower face 2".

Said base element 3 provides a flat lower surface 3" and an upper surface 3', the latter can be removably coupled with the lower face 2" of said central body 2.

Said upper element 4 provides as well an upper surface 4' and a lower surface 4". Said upper surface 4' is flat as well, while lower surface 4" can be removably coupled with said upper surface 2' of said central body 2.

When device 1 is assembled, lower surface 3" of said base element 3 and the upper surface of said upper element 4 lies along not parallel geometric planes, intersecting along a rectilinear line parallel with respect to axis B-B of figure 2. Particularly, it is observed from figure 1 that planes make each other an adjustment angle a.

As it can be observed, plan view of device 1 has a lateral shaping 5, within which, during the use that will be better described in the following, can be placed rear cruciate ligament of the knee.

Central body 2 has a pair of anchoring holes 6 and a channel 7 on its upper face 2' wherein said upper element 4 is housed. Furthermore, on its lateral edge, it is obtained a threaded hole 8. A gripping tool (not shown in the figures) is suitable to enter within said channel 7, coupling with said threaded hole 8 and hooking by said pins within said anchoring holes 6.

Upper element 4 has a substantially "8" shaped shape, permitting:
- following the profile of the lateral shaping 5, when it is juxtaposed with said central body 2;
- letting a portion of said upper face 2' of said central body 2 suitable to the coupling with said tool;
- maintaining symmetry of said device 1 that can be thus used both for balancing of a right or left prosthetic implant.

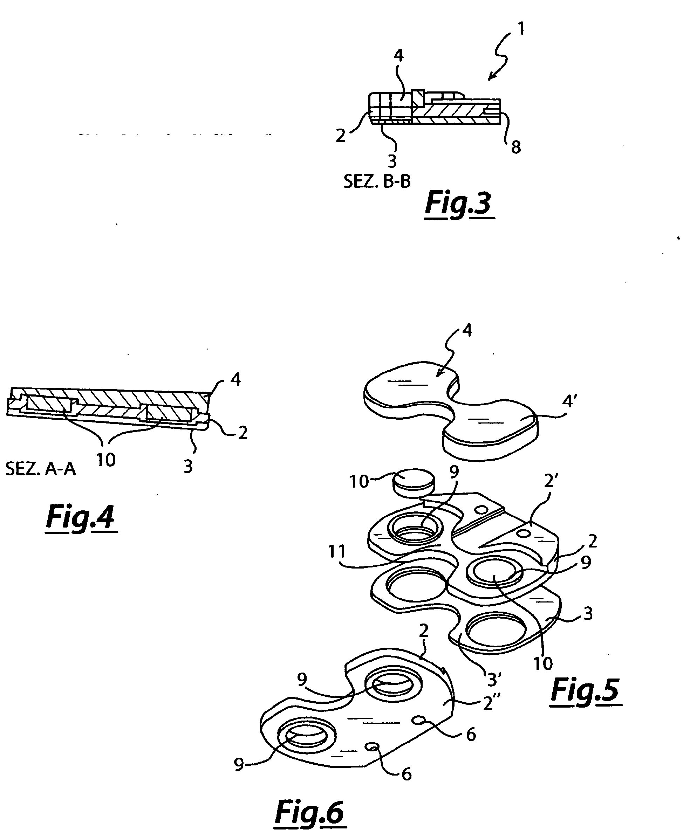

Figures 4, 5 and 6 show in greater detail the inner structure of the device according to the present invention.

Particularly, it is observed that central body 2 provides seats 9 for housing magnets 10. Said magnets 10 are suitable to maintain assembled the device 1 comprised of said central body 2, said base element 3 and said upper element 5, all made up of metal. Obviously, it is possible providing different kind of couplings, such as fixed joint.

Moreover, central body 2 has an upper shaping 11 within which it is possible introducing said upper element 4, so that the latter does not move or rotate when coupled with the first one.

Device 1 according to the invention is realised to be conformable to the different surgery conditions (as it will be exemplified in the following) and to the surgeon preferences. Therefore, device 1 has a plurality of base elements 3, with different thicknesses, and a plurality of different upper elements 4, with different angles or slopes. Thus, surgeon can realise the device 1 with different structural conditions, being it possible realising it by different combinations.

Innovation represented by device 1 according to the invention, is the possibility of the surgeon of evaluating balancing of two basic aspects: parallelism between tibial osteotomy and condyle rear osteotomy, defined on the basis of its own parameters obtained by subjective evaluation through knee varus valgus stress at 90°, evaluation of positioning of rear femur articular interline through its translation along skull-caudal plane compatible with dimensioning of femur component, if necessary intervening, even at the level of front cutting of the size interval between different sizes.

In fact, different inclinations of the upper element 4 define evaluation elements about femur extra-rotation, while base elements 3, with different thicknesses, define positioning of femur articular interline, setting correlation with dimensioning of size of component and its position with respect to the condyle rear plane.

The above can be obtained without interference, conflict or interposition with anatomic structures potentially interfering with evaluation accuracy.

This permit letting to the surgeon freedom of dimensional correspondence or preference of a slight asymmetry of tension under flex-extension or between medial and lateral compartment.

Finally, it must be taken into consideration that said device 1, in a further embodiment, can be realised as a single element, i.e. not in separated parts, presenting flat upper surface and lower surface, inclined each other of adjustment angle a.

Different use mode of device 1 according to the invention will be described in the following.

A first use mode concerns the case in which femur distal and tibial proximal cutting are carried out. In this case, correspondence of space at least with respect to minimum volumetric configuration of the implant (dimensions of the symmetric spacer equivalent to the tibial thickness added to distal femur thickness in the different configurations of tibial thickness) is evaluated by symmetric spacers. Instead, balancing under extension is evaluated stressing articulation under varus-valgus.

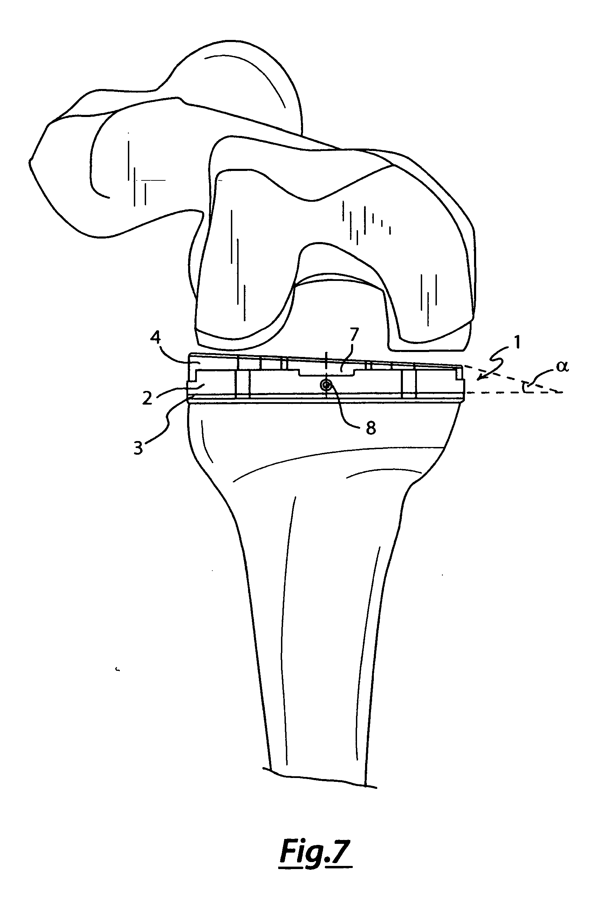

With knee flexed of 90° assembled device 1 is inserted with the following configuration (see also figure 7): central body 2, base element 3, with thickness ranging between 1 and n mm, on the basis of the thickness of the implant under flexion corresponding to the one detected under extension, upper element 4 with an adjustment angle ranging between 1 and n degrees, chosen by the surgeon, on the basis of his preferences.

After having positioned patellar tendon, it is ascertained suitability of the chosen extra-rotation angle and volumetric correspondence of implant with the one detected under extension with varus-valgus stress with a 90° flexion.

In case a stress difference does not exist between medial and lateral compartments, it is varied using upper elements 4 with different angles a and it is repeated check until identifying adjustment angle a suitable to the case.

Once tension symmetry has been considered between medial and lateral compartments, and thus defined femur extra-rotation angle, it is checked the volumetric correspondence defined by base element 3 with stress detected under extension. In case stress is too high, it is checked amount of higher rear condyle cutting necessary to obtain an equivalence increasing flexion space, subtracting to assembling of base element 3 of the device 1, or reducing its thickness.

In case stress is not sufficient, base element 3thickness is increased until identifying thickness necessary and consequently reducing condyle rear cutting closing flexion space.

In case stress is deemed to be suitable, cutting is carried out maintaining level corresponding to the thickness of the femur prosthetic implant.

A second use mode is when surgeon finds a 90° flexion condition, after having carried out a tibial cutting with a thickness corresponding to restoring a proper articular interline.

In this case, device 1 is assembled with the thickness configuration corresponding to the minimum one of prosthetic implant, and then it proceeds as above.

Once set adjustment angle a and condyle rear cutting level, cutting is carried out. Furthermore, it is checked by a base element 3 having dimensions corresponding to the sum of femur and tibial thickness, balancing and correspondence to the different tibial thickness hypothesis.

Then, knee is extended and assembled device 1 is inserted with configuration corresponding to tibial thickness, acting of base element 3. Then, upper element 4 is varied up to identifying condyle distal cutting angle a suitable to obtain varus-valgus balancing of articulation.

In case stress is too high, base element 3 is reduced and at the same time amount of distal femur cutting is increased in a corresponding amount.

In case stress is not sufficient, instead, base element 3 is increased and amount of distal femur cutting is reduced in a corresponding amount. Instead, in case stress is suitable, a femur distal cutting is carried out, corresponding to the femur cutting.

In the second use mode, device 1 can provide evaluation elements relevant to condyle distal cutting angle, defining parallelism between the latter and tibial proximal cutting already realised.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Device (1) for balancing a prosthetic implant for a patient comprising:
a plate-shaped central body (2) having an upper side (2') and a lower side (2"),
a base element (3) having an upper surface (3') removably couplable with said lower side (2") of said central body (2), and a flat lower surface (3"),
a plurality of upper elements (4) having a lower surface (4") removably couplable with said upper side (2') of said central body (2), and an flat upper surface (4'), and
coupling means (10) for coupling said central body (2), said base element (3) and said upper element (4),
**characterized**
**in that** the whole arrangement of said device (1) being such that said upper surface (4') of said upper element (4) is tilted with respect to said flat lower surface (3") of said base element (3) to a preset adjustment angle (a), wherein each of the plurality of upper elements (4) has said flat upper surface (4') tilted to a respective preset adjustment angle (a) with respect to said flat lower surface (3") of said base element (3), and
**in that** the device (1) comprises a plurality of base elements (3) each one having a different thickness.

2. Device (1) according to claim 1, **characterized in that** it provides laterally a shaped portion (5), wherein the rear cruciate ligament of a knee of said patient is placeable.

3. Device according to anyone of the preceding claims, **characterized in that** said central body (2) is provided with one or more seat (9) and said coupling means comprise one or more magnets (10), that can be housed in said seats (9), for maintaining coupled the assembly comprised of said base element (3), said central body (2) and said upper element (4).

4. Device according to anyone of the preceding claims, **characterized in that** said central body (2) has a channel (7) and a pair of anchoring holes (6) placed on said upper side (2'), and a threaded hole (8), placed on the lateral rim, so that a grasping tool provided with pins can be inserted in said channel (7), coupled to said threaded hole (8) and hooked to said anchoring holes (6) by means of said pins.

5. Device according to anyone of the preceding claims, **characterized in that** said upper element (4) is made in such way that said upper surface (4') is tilted with respect to said lower surface (4") to said adjustment angle (a).

6. Device according to anyone of the preceding claims, **characterized in that** said upper element (4) is plate-shaped and has an "8"- shape.

7. Device according to anyone of the preceding claims, **characterized in that** said central body (2) and said base element (3) have substantially the same surface extent.

8. Device according to anyone of the preceding claims, **characterized in that** said adjustment angle (a) is comprised between 0° and 9°.

9. Kit comprising a device according to anyone of the preceding claims, **characterized in that** it comprises a grasping tool provided with pins, said grasping tool being couplable with said central element (2) by insertion in said channel (7), by screwing with said threaded hole (8) and by insertion of said pins in said anchoring holes (6).

## Patentansprüche

1. Vorrichtung (1) zum Abgleichen eines prothetischen Implantats für einen Patienten mit:
einem plattenförmigen Zentral-Körper (2), der eine Oberseite (2') und eine Unterseite (2") aufweist,
einem Grundelement (3), das eine obere Fläche (3'), entfernbar kuppelfähig mit der Unterseite (2") des Zentral-Körpers (2), und eine ebene untere Fläche (3") aufweist, einer Vielzahl oberer Elemente (4), die eine untere Fläche (4"), entfernbar kuppelfähig mit der Oberseite (2') des Zentral-Körpers (2) und eine ebene obere Fläche (4') aufweisen, und
Kupplungs-Mittel (10) zum Kuppeln des Zentral-Körpers (2), des Grundelements (3) und des oberen Elements (4),
**dadurch gekennzeichnet,**
**dass** die gesamte Anordnung der Vorrichtung (1) dergestalt ist, dass die obere Fläche (4') des oberen Elements (4) bezüglich der ebenen unteren Fläche (3") des Grundelements (3) zu einem voreingestellten Abgleichwinkel (a) gekippt ist, wobei jedes der Vielzahl oberer Elemente (4) die ebene obere Fläche (4') zu einem jeweiligen voreingestellten Abgleichwinkel (a) bezüglich der ebenen unteren Fläche (3") des Grundelements (3) gekippt aufweist, und
**dass** die Vorrichtung (1) eine Vielzahl von Grundelementen (3) jeweils mit einer anderen Dicke umfasst.

2. Vorrichtung (1) nach Anspruch 1, **dadurch gekennzeichnet, dass** sie seitlich ein Formteil (5) vorsieht, worin das rückwärtige Kreuzband eines Knies des Patienten anordenbar ist.

3. Vorrichtung nach einem der vorangehenden Ansprüche, **dadurch gekennzeichnet, dass** der Zentral-Körper (2) versehen ist mit einem oder mehreren Sitz(en) (9) und das Kupplungs-Mittel einen oder mehrere Magnete (10) umfasst, die in den Sitzen (9) untergebracht sein können, zum Aufrechtrehalten einer Kupplung der Baugruppe, die das Grundelement (3), den Zentral-Körper (2) und das obere Element (4) umfasst.

4. Vorrichtung nach einem der vorangehenden Ansprüche, **dadurch gekennzeichnet, dass** der Zentral-Körper (2) aufweist einen Kanal (7) und ein Paar von Ankerlöchern (6), angeordnet an der Oberseite (2'), und eine Gewindebohrung (8), angeordnet am seitlichen Rand, so dass ein mit Stiften versehenes Greifwerkzeug in den Kanal (7) eingeschoben werden kann, gekuppelt an die Gewindebohrung (8) und verhakt mit den Ankerlöchern (6) mit Hilfe der Stifte.

5. Vorrichtung nach einem der vorangehenden Ansprüche, **dadurch gekennzeichnet, dass** das obere Element (4) in einer solchen Weise gefertigt ist, dass die obere Fläche (4') bezüglich der unteren Fläche (4") mit dem Abgleichwinkel (a) gekippt ist.

6. Vorrichtung nach einem der vorangehenden Ansprüche, **dadurch gekennzeichnet, dass** das obere Element (4) plattenförmig ist und eine "8"- Form aufweist.

7. Vorrichtung nach einem der vorangehenden Ansprüche, **dadurch gekennzeichnet, dass** der Zentral-Körper (2) und das Grundelement (3) im Wesentlichen dasselbe Flächenausmaß aufweisen.

8. Vorrichtung nach einem der vorangehenden Ansprüche, **dadurch gekennzeichnet, dass** der Abgleichwinkel (a) zwischen 0° und 9° liegt.

9. Kit, umfassend eine Vorrichtung nach einem der vorangehenden Ansprüche, **dadurch gekennzeichnet, dass** es ein mit Stiften versehenes Greifwerkzeug umfasst, wobei das Greifwerkzeug mit dem Zentral-Element (2) durch Einschieben in den Kanal (7), durch Verschrauben mit der Gewindebohrung (8) und durch Einschieben der Stifte in die Ankerlöcher (6) kuppelfähig ist.

## Revendications

1. Dispositif (1) pour équilibrer un implant prothétique pour un patient comprenant :
un corps central en forme de plaque (2) ayant un côté supérieur (2') et un côté inférieur (2"),
un élément de base (3) ayant une surface supérieure (3') pouvant être couplée de manière amovible avec ledit côté inférieur (2") dudit corps central (2), et une surface inférieure plate (3"),
une pluralité d'éléments supérieurs (4) ayant une surface inférieure (4") pouvant être couplée de façon amovible avec ledit côté supérieur (2') dudit corps central (2), et une surface supérieure plate (4'), et
un moyen de couplage (10) pour coupler ledit corps central (2), ledit élément de base (3) et ledit élément supérieur (4),
**caractérisé**
**en ce que** l'arrangement entier dudit dispositif (1) étant tel que ladite surface supérieure (4') dudit élément supérieur (4) est inclinée par rapport à ladite surface inférieure plate (3") dudit élément de base (3) avec un angle d'ajustement prédéfini (a), dans lequel chacun de la pluralité d'éléments supérieurs (4) ont ladite surface supérieure plate (4') inclinée avec respectivement un angle d'ajustement prédéfini (a) par rapport à ladite surface inférieure plate (3") dudit élément de base (3), et
**en ce que** le dispositif (1) comprend une pluralité d'éléments de base (3) chacun ayant une épaisseur différente.

2. Dispositif (1) selon la revendication 1, **caractérisé en ce qu'**il fournit latéralement une portion formée (5), dans lequel le ligament croisé arrière d'un genou dudit patient peut être placé.

3. Dispositif selon l'une quelconque des revendications précédentes, **caractérisé en ce que** ledit corps central (2) est pourvu d'un ou plusieurs sièges (9) et ledit moyen couplant comprend un ou plusieurs aimants (10), qui peuvent être logés dans lesdits sièges (9), pour maintenir couplé l'assemblage comprenant ledit élément de base (3), ledit corps central (2) et ledit élément supérieur (4).

4. Dispositif selon l'une quelconque des revendication précédentes, **caractérisé en ce que** ledit corps central (2) a un canal (7) et une paire de trous d'ancrage (6) placée sur ledit côté supérieur (2'), et un trou taraudé (8), placé sur le rebord latéral, de sorte qu'un outil de préhension pourvu d'épingles peut être inséré dans ledit canal (7), couplé au dit trou taraudé (8) et accroché auxdits trous d'ancrage (6) au moyen desdites épingles.

5. Dispositif selon l'une quelconque des revendications précédentes, **caractérisé en ce que** ledit élément supérieur (4) est fabriqué de telle sorte que ladite surface supérieure (4') est inclinée par rapport à ladite surface inférieure (4") avec ledit angle d'ajustement (a).

6. Dispositif selon l'une quelconque des revendications précédentes, **caractérisé en ce que** ledit élément supérieur (4) a une forme en plaque et a une forme en «8 ».

7. Dispositif selon l'une quelconque des revendications précédentes, **caractérisé en ce que** ledit corps central (2) et ledit élément de base (3) ont essentiellement la même étendue de surface.

8. Dispositif selon l'une quelconque des revendications précédentes, **caractérisé en ce que** ledit angle d'ajustement (a) est compris entre 0° et 9°.

9. Kit comprenant un dispositif selon l'une quelconque des revendications précédentes, **caractérisé en ce qu'**il comprend un outil de préhension pourvu d'épingles, ledit outil de préhension pouvant être étant couplé avec ledit élément central (2) par insertion dans ledit canal (7), en vissant dans ledit trou taraudé (8) et par insertion desdites épingles dans lesdits trous d'ancrage (6).
